# EUROPEAN PATENT SPECIFICATION

(11) **EP 1 860 963 B1**
(45) Date of publication and mention of the grant of the patent: **23.06.2010**
(21) Application number: 06709844.2
(22) Date of filing: 21.02.2006
(51) Int. Cl.: A41D 19/00, A61B 19/04

(54) **GLOVE WITH ANTI-ROLL DOWN CUFF**
HANDSCHUH MIT SICH NICHT HERUNTERROLLENDER STULPE
GANT À POIGNET CONTRE LE GLISSEMENT VERS LE BAS

(30) Priority: 22.03.2005 GB 0505862; 13.06.2005 GB 0511993
(43) Date of publication of application: 05.12.2007
(60) Divisional application: 09170926.1
(73) Proprietor: Regent Medical Limited, Irlam, Manchester M44 5BJ (GB)
(72) Inventor: DAY, Jonathan Regent Medical Limited, Manchester M44 5BJ (GB); LEEMING, Christine Regent Medical Limited, Manchester M44 5BJ (GB); ROGERS, Jan Regent Medical Limited, Manchester M44 5BJ (GB); WILLIAMS, Haydn, Manchester M44 5BJ (GB)
(74) Representative: Hedges, Martin Nicholas
(86) International application number: PCT/GB2006/000611
(87) International publication number: WO 2006/100422

(56) References cited:
- EP-A- 0 359 152
- WO-A-2005/102086
- GB-A- 1 132 599
- US-A- 2 806 257
- US-A- 3 555 564
- US-A- 4 095 293
- US-A1- 2005 229 287
- US-B1- 6 530 090

## Description

The present invention relates to surgical gloves and the like and in particular to such gloves including anti roll down measures provided on the cuff of the glove.

Surgical gloves are designed with a smooth / slippery inner surface with the aim of making it easier for the wearer to don the glove. The gloves are designed to be worn over surgical gowns which were typically cotton based, but recently there have been significant advancements in the material for such gowns, including improvement in the impermeability of the materials achieved by treating the material with a hydrophobic moiety that improves the moisture repellent characteristics of the material. The material from which surgical gloves are typically made has a tacky, adhesive quality, and in order to facilitate the fitting and removal of the gloves onto and from the hands of the user, the formed glove is treated with a material which smoothes the surface thereof. However, an ongoing problem with existing glove and gown designs is that the smooth surfaces of the gloves give rise to a tendency for the cuff of the glove to move down the gown during use, exposing the woven cuff of the gown and hence presenting a risk of strike through of potentially infectious material.

In order to overcome this problem, gloves have been designed having textured wrist portions which are moulded into the material of the glove during the glove moulding process. For example, US-A-4095293 discloses a moulded glove having a plurality of longitudinal channels moulded around the circumference of the wrist portion of the glove together with a plurality of circumferential grooves in the region of the mouth of the glove which operate to prevent roll down. However, this arrangement has the problem that the moulding of the grooves into the glove causing a thinning of material around the wrist and cuff Furthermore, due to the manner in which the grooves are moulded and the fact that the glove is turned inside out upon removal from the former, the grooves will end up on the outside of the glove and the glove hence needs to be reinverted prior to use in order for the grooves to perform their gripping function.

EP-A-0359152, upon which the precharacterising portion of claim 1 is based, discloses a glove comprising a hand portion and a wrist portion extending from the hand portion and terminating in a hand insertion opening, the wrist portion including a region of adhesive material provided on a surface which, in use, will form the inside of the glove.

A glove comprising a hand portion and a wrist portion extending from the hand portion and terminating in a hand insertion opening, the wrist portion including a region of adhesive material provided on a surface which, in use, will form the inside of the glove, **characterised in that** the adhesive material is formed by an unfinished region of glove material, whereby, in use, the adhesive portion is in contact with the cuff of a garment worn by the user so as to prevent roll-down of the glove.

A glove in accordance with the invention has the advantage that it provides an effective and reliable system for preventing roll down of the wrist portion of surgical gloves in a manner which also provides an additional barrier to prevent the ingress of matter between the wrist portion of the glove and the outer surface of the surgical gown.

Preferable, the adhesive material extends around the inner circumference of the wrist portion of the glove which may be in a band, so as, in use, to secure the glove to the cuff of the garment around the entire periphery thereof. This has the advantage of anchoring the glove to the garment in a particularly secure and effective manner.

The adhesive may be a pressure sensitive adhesive that displays a surface of moderate tack or no tack prior to its activation. The adhesive may also be microencapsulated. Once the glove is donned over the gown, the user applies pressure around the cuff area, which causes activation of the adhesive agent, thereby rendering the cuff area tacky to an extent which enables adhesion of the gown.

The adhesive itself may be overlaid with a specific liner or release material which protects and adhesive, preventing it from sticking until required and from becoming polluted with dust, dirt etc, which might otherwise diminish its adhesiveness, until ready for use whilst having a low adhesion to the adhesive so as to be easily removable therefrom. The liner is then removed by the user once the glove has been fitted so as to expose the adhesive for fastening to the cuff of the gown. The liner material advantageously has identical or similar elastomeric properties to those of the glove so that it expands and contracts with the glove.

In the invention, the adhesive strip is formed by an un-finished region of glove material whose tackiness has been maintained. In particular, an adhesive tape may be applied to an exposed region of un-finished glove material, so as to mask the underlying portion of the glove which is still tacky following the forming or moulding operation. The exposed areas of the glove are then finished to provide it with a less tacky surface - adhesive tape maintaining the tackiness of the underlying portion of the glove by preventing its exposure to the finishing material. Subsequent removal of the adhesive tape thus exposes a portion of the glove that has been shielded from the finishing operation and its tackiness is thereby maintained.

A method of manufacture of a surgical glove comprising the steps of coating a former with material to form the glove, applying masking material to the surface of the glove in the region of a wrist portion (2) thereof, and carrying out a finishing operation on the glove in order to produce a smooth surface thereof, the masking material shielding the underlying glove material from the finishing operation so as to maintain the tacky and/or adhesive characteristic thereof.

Preferably the masking or adhesive material is applied in a band which extends around the entire circumference of the wrist portion and is located on a surface which, in use, forms an inside surface of the glove, the glove, in use, overlapping the cuff of the gown on the outside.

In order that the invention may be well understood, there will now be described some embodiment thereof, given by way of example, reference being made to the accompanying drawings, in which:

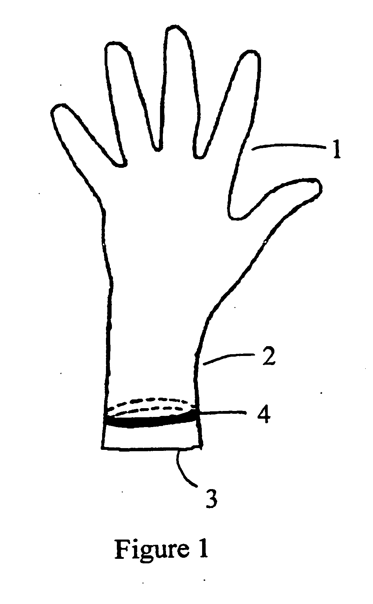

Figure 1 is a perspective view of a surgical glove according to a first embodiment of the invention;

A surgical glove according to the invention as shown in Figure 1 comprises integrally formed hand 1 and cuff 2 portions, the cuff portion terminating in a hand opening 3 by means of which a user may insert a hand into the glove. The glove is manufactured according to normal procedures familiar to those experienced in the art using a former or mould onto which elastomeric material, such as latex, is straight or coagulant dipped and dried to form a film which is subsequently peeled off of the mould. Provided on the cuff portion 2 of the glove is a band of adhesive material 4 which extends around the circumference of the cuff 2, substantially parallel and proximate to the hand opening 3.

In the invention, the band of adhesive material is formed from the material of the glove itself by applying to the tacky surface of the formed but unfinished glove, in the region of the hand opening, a circumferential band of adhesive tape. This band of adhesive tape operates to mask the underlying portion of the glove during a subsequent coating process whereby a suitable slip coating is applied to the glove and / or during an operation in which the surfaces of the glove are treated, for example chemically and / or with a non powdered material the result of which produces a smooth finish thereon. By masking the underlying band of glove material, the tackiness of this portion of material is maintained since it not subjected to the coating or finishing processes. The adhesive tape then protects the underlying portion of material until the glove is ready for use, whereupon the user fits the glove onto the hand with the adhesive tape on the inner surface and the cuff portion positioned to overlap the outer surface of the cuff of the gown worn by the user. The adhesive tape is then removed by the user so as to expose the underlying tacky glove material, which is then pressed against the outer surface of the gown, adhering thereto and hence securing the cuff of the glove in position in a manner which prevents roll-down thereof.

## Claims

1. A glove comprising a hand portion (1) and a wrist portion (2) extending from the hand portion (1) and terminating in a hand insertion (3) opening, the wrist portion (2) including a region of adhesive material (4) provided on a surface which, in use, will form the inside of the glove, **characterised in that** the adhesive material (4) is formed by an unfinished region of glove material, whereby, in use, the adhesive portion (4) is in contact with the cuff of a garment worn by the user so as to prevent roll-down of the glove.

2. A glove according to claim 1 which is a surgical glove.

3. A glove according to claim 1 or claim 2, wherein the region of adhesive material (4) extends entirely around the inner circumference of the wrist portion (2) of the glove.

4. A glove according to any of the preceding claims, wherein the adhesive is a pressure sensitive adhesive which is activated, in use, by application of pressure to the surface thereof.

5. A glove according to any of the preceding claims, wherein the adhesive material (4) is overlaid with a strip of material which masks the underlying region of the glove during a glove finishing process, thereby maintaining the tackiness of the underlying glove material, and which also protects the underlying material from pollution prior to use.

6. A glove according to claim 5, wherein the strip of material is release material.

7. A glove according to claim 5 or claim 6, wherein the strip of material has elastomeric properties which are substantially the same as the elastomeric properties of the material of the glove such that the strip of material stretches and contracts with the underlying glove material.

8. A method of manufacture of a surgical glove comprising the steps of coating a former with material to form the glove, applying masking material to the surface of the glove in the region of a wrist portion (2) thereof, and carrying out a finishing operation on the glove in order to produce a smooth surface thereof, the masking material shielding the underlying glove material from the finishing operation so as to maintain the tacky and/or adhesive characteristic thereof.

9. A method according to claim 8, wherein the finishing operation comprises applying a non-powdered coating to the surface of the glove.

10. A method according to claim 8 or claim 9, wherein the masking or adhesive material is applied in a band (4) which extends around the entire circumference of the wrist portion (2).

11. A method according to any of claims 8 to 10, wherein the masking or adhesive material (4) is applied to a surface of the glove which, in use, forms an inside surface of the glove.

## Patentansprüche

1. Handschuh, enthaltend einen Handabschnitt (1) und einen Handgelenksabschnitt (2), der sich von dem Handabschnitt (1) erstreckt und in einer Handeinführöffnung (3) endet, wobei der Handgelenksabschnitt (2) einen Bereich eines Haftmaterials (4) enthält, das auf einer Oberfläche vorgesehen ist, die in Benutzung die Innenseite des Handschuhs bildet, **dadurch gekennzeichnet, dass** das Haftmaterial (4) durch einen nicht fertig bearbeiteten Bereich des Handschuhmaterials ausgebildet ist, wobei in Benutzung der Haftabschnitt (4) mit dem Bund eines Kleidungsstückes in Kontakt ist, das vom Benutzer getragen wird, so dass ein Herunterrollen des Handschuhs verhindert wird.

2. Handschuh nach Anspruch 1, der ein chirurgischer Handschuh ist.

3. Handschuh nach Anspruch 1 oder 2, bei dem sich der Bereich des Haftmaterials (4) vollständig um den Innenumfang des Handgelenkabschnittes (2) des Handschuhs erstreckt.

4. Handschuh nach einem der vorhergehenden Ansprüche, bei dem das Haftmittel ein druckempfindliches Haftmittel ist, das bei Benutzung durch Druck auf dessen Oberfläche aktiviert wird.

5. Handschuh nach einem der vorhergehenden Ansprüche, bei dem das Haftmaterial (4) von einem Streifen eines Materials bedeckt ist, das den darunter liegenden Bereich des Handschuhs während eines Handschuh-Endbearbeitungsvorgangs maskiert, wodurch die Klebkraft des drunter liegenden Handschuhmaterials erhalten bleibt, und das zudem das darunter liegende Material vor einer Verschmutzung vor der Verwendung bewahrt.

6. Handschuh nach Anspruch 5, bei dem der Materialstreifen aus einem lösbaren Material besteht.

7. Handschuh nach Anspruch 5 oder 6, bei dem der Materialstreifen elastomere Eigenschaften hat, die im wesentlichen dieselben sind wie die elastomeren Eigenschaften des Materials des Handschuhs, so dass sich der Materialstreifen mit dem darunter liegenden Handschuhmaterial ausdehnt und zusammenzieht.

8. Verfahren zum Herstellen eines chirurgischen Handschuhs, umfassend folgende Schritte: Beschichten eines Formteils mit einem Material zum Ausbilden des Handschuhs, Auftragen eines Maskiermaterials auf die Oberfläche des Handschuhs in einem Bereich eines Handgelenkabschnittes (2) desselben und Ausführen eines Fertigbearbeitungsvorgangs an dem Handschuh, um eine glatte Oberfläche desselben zu erzeugen, wobei das Maskiermaterial das darunter liegende Handschuhmaterial von dem Fertigbearbeitungsvorgang abschirmt, um so die klebrigen und/oder haftenden Eigenschaften desselben zu erhalten.

9. Verfahren nach Anspruch 8, bei dem der Fertigbearbeitungsvorgang das Auftragen einer nicht gepulverten Beschichtung auf die Oberfläche des Handschuhs beinhaltet.

10. Verfahren nach Anspruch 8 oder 9, bei dem das maskierende oder das haftende Material in einem Band (4) aufgebracht wird, das sich um den gesamten Umfang des Handgelenkabschnittes (2) erstreckt.

11. Verfahren nach einem der Ansprüche 8 bis 10, bei dem das maskierende oder haftende Material (4) auf eine Oberfläche des Handschuhs aufgebracht wird, die in Benutzung eine Innenoberfläche des Handschuhs bildet.

## Revendications

1. Gant comprenant une partie de main (1) et une partie de poignet (2) s'étendant à partir de la partie de main (1) et se terminant dans une ouverture d'insertion de main (3), la partie de poignet (2) comprenant une région de matériau adhésif (4) prévue sur une surface qui, en utilisation, va former l'intérieur du gant, **caractérisé en ce que** le matériau adhésif (4) est formé par une région non finie de matériau de gant, grâce à quoi, en utilisation, la partie adhésive (4) est en contact avec le poignet d'un vêtement porté par l'utilisateur de manière à empêcher le glissement du gant.

2. Gant selon la revendication 1 qui est un gant chirurgical.

3. Gant selon la revendication 1 ou la revendication 2, où la région de matériau adhésif (4) s'étend entièrement autour de la circonférence intérieure de la partie de poignet (2) du gant.

4. Gant selon l'une quelconque des revendications précédentes, où l'adhésif est un adhésif sensible à la pression qui est activé, en utilisation, par l'application d'une pression à sa surface.

5. Gant selon l'une quelconque des revendications précédentes, où le matériau adhésif (4) est revêtu d'une bande de matériau qui masque la région sous-jacente du gant durant un procédé de finition du gant, en maintenant ainsi l'adhésivité du matériau de gant sous-jacent, et qui protège également le matériau sous-jacent de la pollution avant utilisation.

6. Gant selon la revendication 5, où la bande de matériau est un matériau libérable.

7. Gant selon la revendication 5 ou la revendication 6, où la bande de matériau possède des propriétés élastomères qui sont sensiblement identiques aux propriétés élastomères du matériau du gant de sorte que la bande de matériau s'étire et se contracte avec le matériau de gant sous-jacent.

8. Procédé de fabrication d'un gant chirurgical comprenant les étapes consistant à revêtir une forme avec un matériau pour former le gant, appliquer un matériau de masquage à la surface du gant dans la région d'une partie de poignet (2) de celui-ci, et réaliser une opération de finition sur le gant afin de produire une surface lisse pour celui-ci, le matériau de masquage protégeant le matériau de gant sous-jacent de l'opération de finition de manière à maintenir ses caractéristiques collante et/ou adhésive.

9. Procédé selon la revendication 8, où l'opération de finition consiste à appliquer un revêtement non pulvérulent à la surface du gant.

10. Procédé selon la revendication 8 ou la revendication 9, où le matériau de masquage ou adhésif est appliqué dans une bande (4) qui s'étend autour de la circonférence complète de la partie de poignet (2).

11. Procédé selon l'une quelconque de revendication 8 à 10, où le matériau de masquage ou adhésif (4) est appliqué à une surface du gant qui, en utilisation, forme une surface intérieure du gant.
